# EUROPEAN PATENT SPECIFICATION

(11) **EP 4 487 786 B1**
(45) Date of publication and mention of the grant of the patent: **24.06.2026**
(21) Application number: 23779288.2
(22) Date of filing: 07.03.2023
(51) Int. Cl.: A61B 8/12, A61B 5/06

(54) **COMPUTER PROGRAM, INFORMATION PROCESSING DEVICE, AND INFORMATION PROCESSING METHOD**
COMPUTERPROGRAMM, INFORMATIONSVERARBEITUNGSVORRICHTUNG UND INFORMATIONSVERARBEITUNGSVERFAHREN
PROGRAMME INFORMATIQUE, DISPOSITIF DE TRAITEMENT D'INFORMATIONS ET PROCÉDÉ DE TRAITEMENT D'INFORMATIONS

(30) Priority: 28.03.2022 JP 2022052276
(43) Date of publication of application: 08.01.2025
(73) Proprietor: Terumo Kabushiki Kaisha, Tokyo 151-0072 (JP)
(72) Inventor: TOMINAGA, Takanori, Ashigarakami-gun, Kanagawa 259-0151 (JP); SAKAGUCHI, Yuki, Ashigarakami-gun, Kanagawa 259-0151 (JP)
(74) Representative: Casalonga
(86) International application number: PCT/JP2023/008439
(87) International publication number: WO 2023/189260

(56) References cited:
- EP-B1- 3 376 942
- WO-A1-2020/257619
- WO-A1-2020/257619
- JP-A- 2011 200 596
- JP-A- 2018 535 765
- JP-A- 2020 163 216
- US-A1- 2011 237 958

## Description

### Technical Field

The present invention relates to a computer program, an information processing apparatus, and an information processing method.

### Background Art

Patent Literature 1 discloses an image diagnosis apparatus that generates a blood vessel cross-sectional image on the basis of a signal (ultrasound emitted to a vascular tissue and reflected wave) obtained by an imaging core accommodated in a catheter inserted into a blood vessel.

Under percutaneous coronary intervention (PCI), a sheath is inserted into a blood vessel, a soft wire called a guide wire travels through the sheath along the blood vessel, and a guiding catheter is placed at the entrance of a coronary artery along the guide wire. Then, a device such as a catheter, a stent, or a balloon is inserted into the coronary artery through the guiding catheter. The publications EP 3 376 942 B1, US 2011/237958 A1 and WO 2020/257619 A1 disclose prior art methods and systems for guiding catheter identification.

### Citation List

### Patent Literature

Patent Literature 1: WO 2017/164071 A

### Summary of Invention

### Technical Problem

When a catheter is inserted into a blood vessel and generates a medical image by causing a sensor provided at a distal end of the catheter to measure a cross-section of the blood vessel, a pull-back operation of moving the sensor from a distal end (distal) side to a proximal end (proximal) side while rotating the sensor in a circumferential direction is made. As the location of observation by the sensor progresses into the guiding catheter from a blood vessel where no guiding catheter is present, the inside of the guiding catheter may be misidentified as a lumen. Therefore, if the position of the guiding catheter is unknown, the position, diameter, area, or the like of a stenosis cannot be correctly evaluated.

The present invention has been made in view of such circumstances, and it is therefore an object of the present invention to provide a computer program, an information processing apparatus, and an information processing method capable of determining the position of a guiding catheter.

### Solution to Problem

The present application includes a plurality of means for solving the above-described problems, and examples of the means includes a computer program causing a computer to execute processing of: acquiring medical image data representing a plurality of frames of cross-sectional images of a luminal organ into which a guiding catheter has been inserted; identifying a guiding catheter determination region for determining the guiding catheter on the basis of target frames of cross-sectional images that are all or some of the plurality of frames of the acquired medical image data; and determining whether or not the guiding catheter is present in each of the target frames on the basis of pixel values of the identified guiding catheter determination region.

### Advantageous Effects of Invention

According to the present invention, it is possible to determine the position of the guiding catheter.

### Brief Description of Drawings

Fig. 1 is a diagram illustrating an example of a configuration of an information processing system according to the present embodiment.
Fig. 2 is a diagram illustrating an example of a configuration of an information processing apparatus.
Fig. 3 is a diagram illustrating an example of medical image data.
Fig. 4 is a diagram illustrating an example of a cross-sectional image of a blood vessel into which a guiding catheter has been inserted.
Fig. 5 is a diagram illustrating a first example of a guiding catheter determination region.
Fig. 6 is a diagram illustrating an example of a method of determining a guiding catheter.
Fig. 7 is a diagram illustrating a first example of a method of identifying an inner perimeter boundary.
Fig. 8 is a diagram illustrating a second example of the method of identifying an inner perimeter boundary.
Fig. 9 is a diagram illustrating a second example of the guiding catheter determination region.
Fig. 10 is a diagram illustrating a guiding catheter and a calcified plaque on a cross-sectional image.
Fig. 11 is a diagram illustrating an example of a method of distinguishing between a guiding catheter and a calcified plaque.
Fig. 12 is a diagram illustrating an example of a method of selecting a target frame for higher performance.
Fig. 13 is a diagram illustrating an example of processing based on a learning model.
Fig. 14 is a diagram illustrating an example of segmentation data output by the learning model.
Fig. 15A is a diagram illustrating a comparative example in which a guiding catheter is misidentified as a lumen of a blood vessel.
Fig. 15B is a diagram illustrating an example of detecting a guiding catheter according to the present embodiment.
Fig. 16A is a diagram illustrating an example of blood vessel information obtained by the information processing apparatus.
Fig. 16B is a diagram illustrating an example of the blood vessel information obtained by the information processing apparatus.
Fig. 17 is a diagram illustrating an example of a processing procedure by the information processing apparatus.

### Description of Embodiments

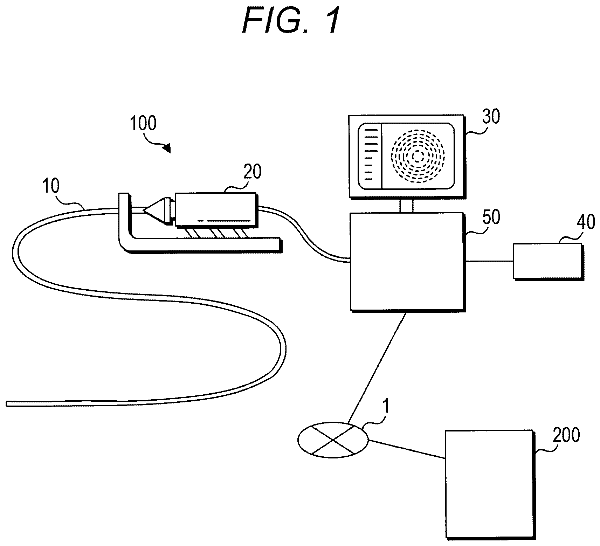

Hereinafter, an embodiment of the present invention will be described. Fig. 1 is a diagram illustrating an example of a configuration of an information processing system 100 according to the present embodiment. The information processing system 100 is, for example, an image diagnosis system and an apparatus for performing intravascular imaging (image diagnosis) used for cardiac catheter treatment (PCI: Percutaneous Coronary Intervention). The cardiac catheter treatment is a method of treating a narrowed portion of a coronary artery by inserting a catheter from a blood vessel such as a base of a leg, an arm, or a wrist. For intravascular imaging, there are two methods: an intra vascular ultra sound (IVUS) method; and an optical coherence tomography (OFDI: Optical Frequency Domain Imaging, OCT: Optical Coherence Tomography) method. The IVUS uses reflection of ultrasound to interpret the inside of a blood vessel in a tomographic image. Specifically, a thin catheter equipped with an ultra-small sensor at its distal end is inserted into a coronary artery, and after passing to a lesion, a medical image representing the inside of a blood vessel can be generated using ultrasound transmitted from the sensor (ultrasound transmitter and receiver). The OFDI uses near-infrared rays to interpret a state in a blood vessel with a high-resolution image. Specifically, as in the IVUS, a catheter is inserted into a blood vessel, near-infrared rays are emitted from its distal end, a cross-section of the blood vessel is measured by interferometry, and a medical image is generated. Furthermore, the OCT is intravascular image diagnosis to which near-infrared rays and optical fiber technology are applied. A blood vessel will be described herein as an example of a luminal organ. Furthermore, the medical image (medical image data) includes one generated by the IVUS, the OFDI, or the OCT, but the case of using the IVUS method will be mainly described herein below.

The information processing system 100 includes a catheter 10, a motor drive unit (MDU) 20, a display apparatus 30, an input apparatus 40, and an information processing apparatus 50. A server 200 is connected to the information processing apparatus 50 via a communication network 1.

The catheter 10 is an image diagnosis catheter for obtaining an ultrasound tomographic image of a blood vessel by the IVUS method. The catheter 10 has an ultrasound probe provided at its distal end for obtaining an ultrasound tomographic image of a blood vessel. The ultrasound probe includes an ultrasound transducer that emits ultrasound in a blood vessel, and an ultrasound sensor that receives a reflected wave (ultrasound echo) reflected by a structure such as a biological tissue of the blood vessel or a medical device. The ultrasound probe is configured to be movable back and forth in a longitudinal direction of the blood vessel while rotating in a circumferential direction of the blood vessel.

The MDU 20 is a drive apparatus to which the catheter 10 can be detachably attached, and drives a built-in motor in response to the operation made by a medical worker to control the motion of the catheter 10 inserted into a blood vessel. The MDU 20 can rotate the ultrasound probe of the catheter 10 in the circumferential direction while moving the ultrasound probe from the distal end (distal) side to the proximal end (proximal) side (pull-back operation). The ultrasound probe continuously scans the inside of the blood vessel at predetermined time intervals, and outputs reflected wave data of the detected ultrasound to the information processing apparatus 50.

The information processing apparatus 50 acquires medical image data representing time-series (a plurality of frames of) cross-sectional images of the blood vessel on the basis of the reflected wave data output from the ultrasound probe of the catheter 10. Since the ultrasound probe scans the inside of the blood vessel while moving from the distal end (distal) side to the proximal end (proximal) side in the blood vessel, a plurality of medical images in chronological order is tomographic images of the blood vessel observed at a plurality of points from the distal side to the proximal side.

The display apparatus 30 includes a liquid crystal display panel, an organic EL display panel, or the like, and can display a result of processing executed by the information processing apparatus 50. Furthermore, the display apparatus 30 can display the medical image generated (acquired) by the information processing apparatus 50.

The input apparatus 40 is an input interface such as a keyboard and a mouse that receives input of various setting values, an operation for the information processing apparatus 50, and the like when an examination is made. The input apparatus 40 may be a touch panel, a software key, a hardware key, or the like provided in the display apparatus 30.

The server 200 is, for example, a data server, and may include an image DB in which the medical image data is accumulated.

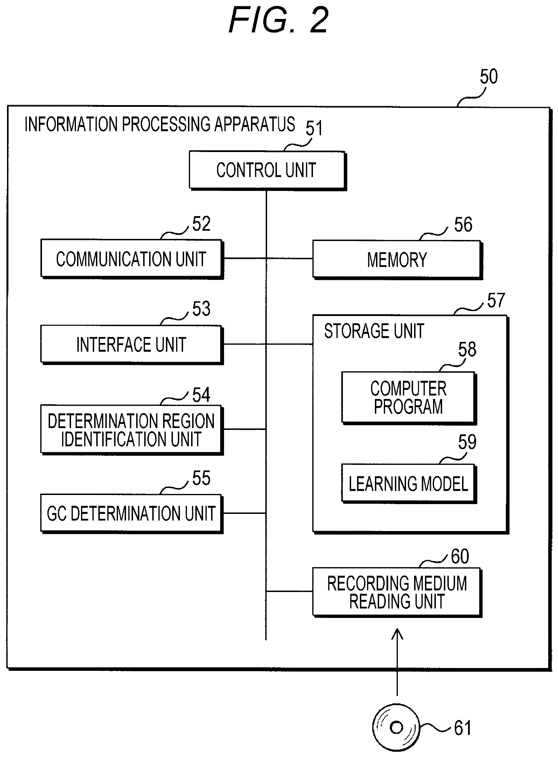

Fig. 2 is a diagram illustrating an example of a configuration of the information processing apparatus 50. The information processing apparatus 50 includes a control unit 51 that controls the entire information processing apparatus 50, a communication unit 52, an interface unit 53, a determination region identification unit 54, a GC determination unit 55, a memory 56, a storage unit 57, and a recording medium reading unit 60. The storage unit 57 stores a computer program 58 and a learning model 59.

The determination region identification unit 54 and the GC determination unit 55 may be implemented by hardware, may be implemented by software (computer program 58), or may be implemented by both hardware and software. The information processing apparatus 50 may include a plurality of information processing apparatuses to distribute its function over the plurality of information processing apparatuses.

The control unit 51 may include a predetermined number of central processing units (CPUs), micro-processing units (MPUs), graphics processing units (GPUs), general-purpose computing on graphics processing units (GPGPUs), or tensor processing units (TPUs). Furthermore, the control unit 51 may include a combination of digital signal processors (DSPs), field-programmable gate arrays (FPGAs), quantum processors, and the like.

The control unit 51 can execute processing defined by the computer program 58. That is, the processing executed by the control unit 51 corresponds to processing executed in accordance with the computer program 58.

The memory 56 may include a semiconductor memory, such as a static random access memory (SRAM), a dynamic random access memory (DRAM), or a flash memory. Loading the computer program 58 into the memory 56 enables the control unit 51 to execute the computer program 58.

The communication unit 52 includes, for example, a communication module and has a capability of communicating with the server 200 over the communication network 1. Furthermore, the communication unit 52 may have a capability of communicating with an external device (not illustrated) connected to the communication network 1. The communication unit 52 may acquire, from the server 200, medical image data representing a plurality of frames of cross-sectional images of a blood vessel into which the guiding catheter has been inserted.

The interface unit 53 provides an interface function between the catheter 10, the display apparatus 30, and the input apparatus 40. The information processing apparatus 50 (control unit 51) can transmit and receive data or information to and from the catheter 10, the display apparatus 30, and the input apparatus 40 via the interface unit 53. The interface unit 53 may acquire, from the catheter 10, medical image data representing a plurality of frames of cross-sectional images of a blood vessel into which the guiding catheter has been inserted.

The guiding catheter (also referred to as "GC") is a catheter for guiding a treatment instrument (device), such as a guide wire, a catheter (for example, an IVUS catheter), a stent, or a balloon, to a coronary artery, and such treatment instruments can pass through the inside of the guiding catheter to reach a lesion.

The recording medium reading unit 60 can include, for example, an optical disc drive, and the computer program 58 (program product) recorded on a recording medium 61 (for example, an optically readable disc storage medium such as a CD-ROM) can be read by the recording medium reading unit 60 and stored into the storage unit 57. The computer program 58 is loaded into the memory 56 and executed by the control unit 51. Note that the computer program 58 may be downloaded from an external device via the communication unit 52 and stored into the storage unit 57.

The storage unit 57 can include, for example, a hard disk, a semiconductor memory, or the like, and can store necessary information (for example, data being processed by the information processing apparatus 50, a processing result, and the like).

The determination region identification unit 54 functions as an identification unit to identify a guiding catheter determination region for determining a guiding catheter on the basis of target frames of cross-sectional images that are all or some of the plurality of frames of medical image data acquired via the interface unit 53 or the communication unit 52. The target frame is a determination target frame that is used for determining whether or not a guiding catheter is present. Details of the method of identifying the guiding catheter determination region will be described later.

The GC determination unit 55 functions as a determination unit to determine whether or not a guiding catheter is present in the target frame on the basis of pixel values of the guiding catheter determination region identified by the determination region identification unit 54. Details of the determination method will be described later.

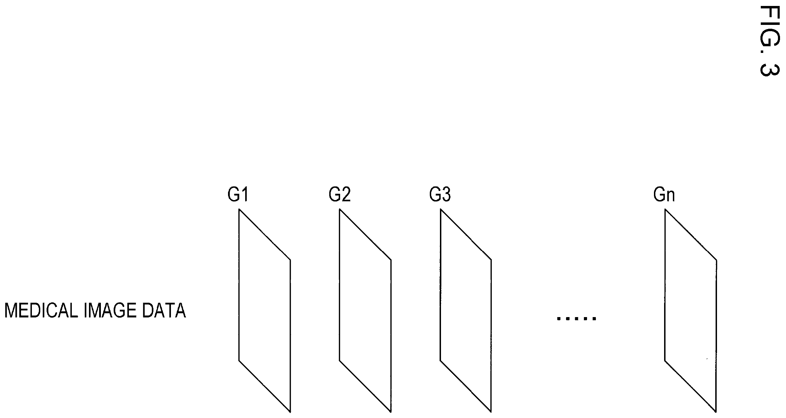

Fig. 3 is a diagram illustrating an example of medical image data. The ultrasound probe provided in the catheter can acquire medical image data (G1, G2, G3, ..., Gn) including a plurality of frames (frames 1 to n) of cross-sectional images as illustrated in Fig. 3 by scanning the inside of a blood vessel while moving from the distal end (distal) side to the proximal end (proximal) side in the blood vessel. In the example in the drawing, the cross-sectional image G1 is image data on the most distal side, and the cross-sectional image Gn is image data on the most proximal side.

Next, the guiding catheter determination region (GC determination region) will be described.

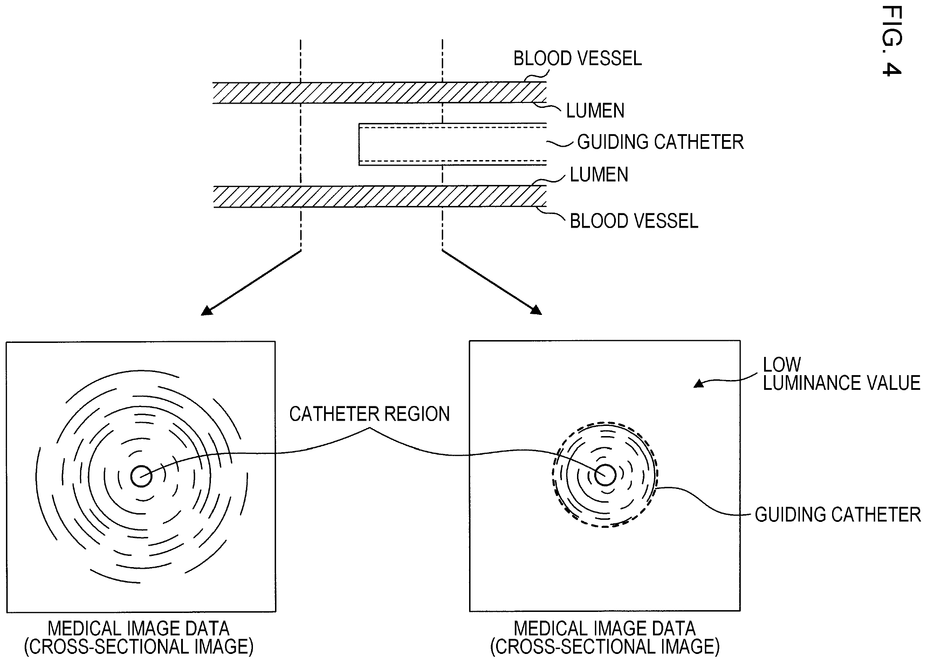

Fig. 4 is a diagram illustrating an example of a cross-sectional image of a blood vessel into which the guiding catheter has been inserted. Fig. 4 schematically illustrates a cross-sectional image of the outside of the guiding catheter and a cross-sectional image of the inside of the guiding catheter. In the cross-sectional image of the outside of the guiding catheter, a relatively bright area (higher in luminance value) and a relatively dark area (lower in luminance value) both appear and are distributed over a perimeter region of a catheter region indicating the position of the catheter according to a state inside the blood vessel. On the other hand, in the cross-sectional image of the inside of the guiding catheter, a relatively bright area (higher in luminance value) and a relatively dark area (lower in luminance value) both appear and are distributed over a region near the catheter region, but a region outside the region near the catheter region is dark as a whole and lower in luminance value. Such a distribution of luminance values is considered to represent a feature of a case where the guiding catheter is present. That is, most of the ultrasound transmitted from the sensor (ultrasound transmitter and receiver) of the catheter is reflected off an inner perimeter surface of the guiding catheter and return to the sensor, so that the ultrasound hardly reaches the region outside the guiding catheter, and as a result, the region outside the guiding catheter becomes lower in luminance value (alternatively, there is no high-luminance pixel). In other words, it is possible to determine the presence or absence of the guiding catheter by measuring the pixel value (for example, the luminance value) of the region outside the guiding catheter.

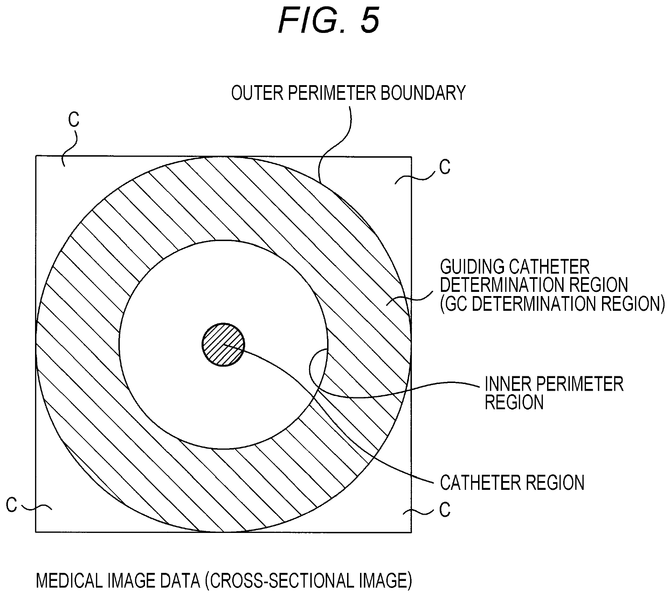

Fig. 5 is a diagram illustrating a first example of the guiding catheter determination region. As illustrated in Fig. 5, the guiding catheter determination region (GC determination region) may include concentric regions surrounding the catheter region and defined by an outer perimeter boundary and an inner perimeter boundary. The determination region identification unit 54 may identify the outer perimeter boundary of the guiding catheter determination region on the basis of the catheter region of the catheter inserted into the guiding catheter. Specifically, on the basis of a distance over which the ultrasound transmitted from the sensor provided in the catheter can propagate, a range from an outer perimeter portion of the catheter region (location where the sensor is attached) to a location at the distance away from the outer perimeter portion can be identified as the outer perimeter boundary. The method of identifying the inner perimeter boundary will be described later.

In a case where a statistic of the pixel values of the guiding catheter determination region in the target frame is less than or equal to a required threshold, the GC determination unit 55 can determine that the guiding catheter is present in the target frame. The statistic may be a mean, a median, or a mode of the pixel values of the pixels of the guiding catheter determination region. The range of the luminance value can be, for example, 0 (dark) to 255 (bright). In a case where the guiding catheter is present in the target frame, the guiding catheter determination region has no high-luminance pixels and includes a set of relatively low-luminance pixels, so that the mean luminance value becomes small, for example.

As illustrated in Fig. 5, it is possible to exclude, by defining the outer perimeter boundary, pixel values of four corner regions indicated by a reference sign C in the rectangular medical image from the determination of the presence or absence of the guiding catheter. As a result, when the GC determination unit 55 determines the presence or absence of the guiding catheter on the basis of the pixel values of the guiding catheter determination region, the statistic of the pixel values of the guiding catheter determination region in the target frame can be prevented from becoming unnecessarily small due to the pixel values of the region indicated by the reference sign C, and the presence or absence of the guiding catheter can be accurately determined.

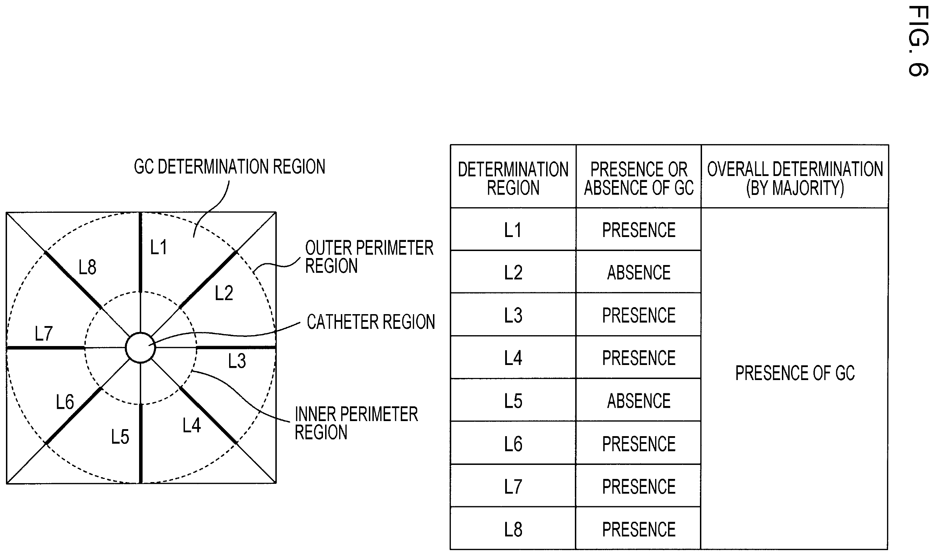

Fig. 6 is a diagram illustrating an example of the method of determining a guiding catheter. As illustrated in Fig. 6, a plurality of virtual line segments extending from a center portion of the catheter region in a radial direction are set. In the example in Fig. 6, line segments L1 to L8 arranged in a circle at intervals of 45° around the center portion of the catheter region are set. The GC determination unit 55 can determine whether or not the guiding catheter is present in the target frame on the basis of the pixel values of the guiding catheter determination region on the plurality of line segments extending, in the radial direction, from the center portion of the catheter region of the catheter inserted into the guiding catheter. In this case, the determination of the guiding catheter is made on the basis of a statistic of the pixel values on the line segments L1 to L8 in the guiding catheter determination region. Specifically, the presence or absence of the guiding catheter is determined for each of the determination regions L1 to L8, and whether or not the guiding catheter is present in the target frame is finally determined on the basis of the determination result for each of the determination regions L1 to L8 under majority rule. In the example in Fig. 6, it is determined that GC is present in six line segments among the eight line segments L1 to L8, and it is determined that no GC is present in the remaining two line segments, so that it can be determined that the guiding catheter is present in the target frame under majority rule.

As described above, it is not necessary to process the pixel values of all the pixels in the guiding catheter determination region, so that it is possible to determine the presence or absence of the guiding catheter quickly. Furthermore, since the final determination is made under majority rule using the respective determination results for the plurality of line segments, the determination accuracy can be improved. Note that the number of line segments is not limited to the example in Fig. 6. The number of line segments may be an odd number.

The required threshold used when the GC determination unit 55 determines the presence or absence of the guiding catheter may be a fixed value or may be set as desired. For example, the GC determination unit 55 may set the required threshold on the basis of distribution of pixel values of an inner region inside the inner perimeter boundary of the guiding catheter determination region (region between the outer perimeter of the catheter region and the inner perimeter boundary). For example, a local minimum of the distribution of the pixel values (for example, the luminance values) of the inner region, an intermediate value between a local maximum and the local minimum, a value obtained by weighting a mean of the pixel values of the inner region with a predetermined value, or the like may be used as the threshold. Note that the method of setting the threshold is not limited to the above. The threshold may be a common threshold for all target frames, or may be a threshold set for each target frame. Similarly, as for the identification of the guiding catheter determination region, the guiding catheter determination region may be a common guiding catheter determination region identified for all target frames, or may be a guiding catheter determination region identified for each target frame.

Next, a method of identifying the inner perimeter boundary will be described.

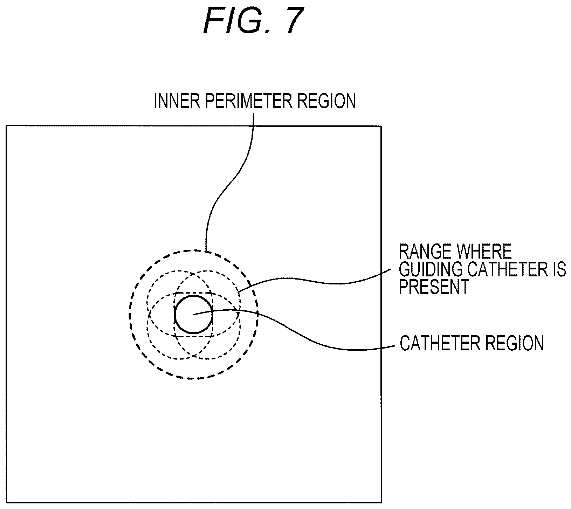

Fig. 7 is a diagram illustrating a first example of the method of identifying the inner perimeter boundary. In Fig. 7, a solid line indicates the catheter region, and a thin dashed line indicates a range where the guiding catheter into which the catheter has been inserted is present, that is, a range where the guiding catheter can move under physical constraints. A diameter of the thin dashed line is determined by a diameter of the guiding catheter. Then, a boundary indicated by a thick dashed line by which the range indicated by the thin dashed line where the guiding catheter is present is enclosed can be identified as the inner perimeter boundary. As described above, the determination region identification unit 54 can identify the inner perimeter boundary of the guiding catheter determination region on the basis of the catheter region of the catheter inserted into the guiding catheter and the diameter of the guiding catheter.

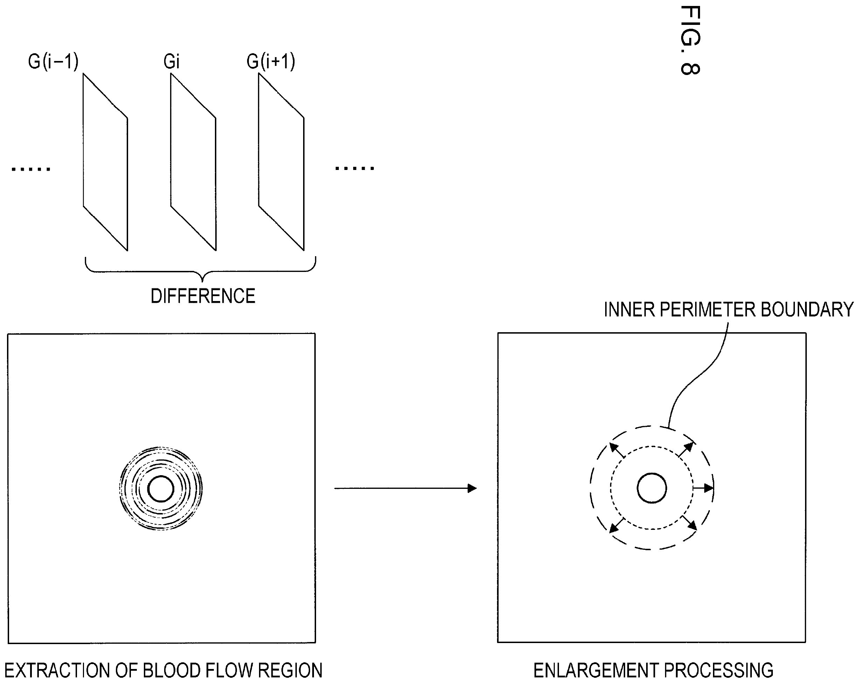

Fig. 8 is a diagram illustrating a second example of the method of identifying the inner perimeter boundary. There is blood flow outside and inside the guiding catheter, and the blood flow outside the guiding catheter can be hardly measured by the sensor of the catheter, but the blood flow inside the guiding catheter can be measured by the sensor. Since the blood flow changes from moment to moment, the pixel value (luminance value) of the same position (same pixel) on the cross-sectional image changes with time. It is therefore possible to identify, by identifying a region where the luminance changes due to the blood flow, the position and shape of the guiding catheter on the cross-sectional image and identify the inner perimeter boundary in a region outside the identified position and shape of the guiding catheter.

As described above, the determination region identification unit 54 can identify a blood flow region on the basis of a difference between the pixel values of corresponding positions on the plurality of target frames of cross-sectional images, and identify the inner perimeter boundary of the guiding catheter determination region on the basis of the identified blood flow region. The plurality of target frames may be at least two or more frames. Fig. 8 illustrates three target frames: a frame (i - 1), a frame (i), and a frame (i + 1). In this case, pixels having a difference in pixel value between the frame (i - 1) and the frame (i) greater than or equal to a predetermined threshold are extracted, and pixels having a difference in pixel value between the frame (i) and the frame (i + 1) greater than or equal to the predetermined threshold value are extracted. The extracted pixels constitute a blood flow region as illustrated in Fig. 8. Then, the determination region identification unit 54 can identify the inner perimeter boundary by executing processing of enlarging the boundary of the extracted blood flow region outward in the radial direction. A scale of enlargement applied to the enlargement processing may be, for example, about several pixels.

It is possible to identify, by identifying the inner perimeter boundary, the guiding catheter determination region in a region outside the guiding catheter, and accurately determine the presence or absence of the guiding catheter.

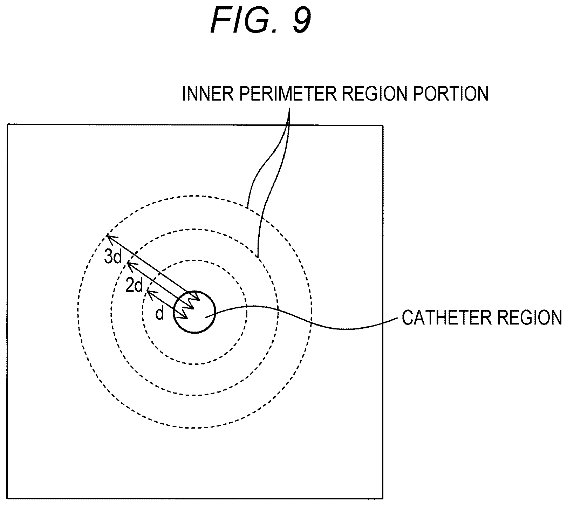

Fig. 9 is a diagram illustrating a second example of the guiding catheter determination region. When the ultrasound transmitted from the sensor provided in the catheter is reflected off the inner perimeter surface of the guiding catheter, multiple reflection may occur in which the reflected wave is reflected again off an outer perimeter surface of the catheter to travel toward the inner perimeter surface of the guiding catheter, and is reflected again off the inner perimeter surface of the guiding catheter. In this case, multiple echoes occur in the region outside the guiding catheter. The multiple echoes occur concentrically with the circular shape of the guiding catheter on the cross-sectional image. Fig. 9 schematically illustrates a portion (inner perimeter region portion) where concentric multiple echoes may occur at positions where a distance from the catheter region is an integral multiple of a distance d (that is, 2d, 3d, and the like), where d is a distance between the catheter region and the guiding catheter.

The determination region identification unit 54 can identify the inner perimeter region portion having a diameter of an integral multiple of the predetermined distance d in the radial direction from the center portion of the catheter region of the catheter inserted into the guiding catheter, and can remove the inner perimeter region portion from the guiding catheter determination region. A width of the inner perimeter region portion in the radial direction may be, for example, several pixels. As a result, it is possible to improve the accuracy of the determination of the presence or absence of the guiding catheter by removing a region having relatively high luminance generated by multiple echoes.

Next, a method of distinguishing between the guiding catheter and a calcified plaque will be described.

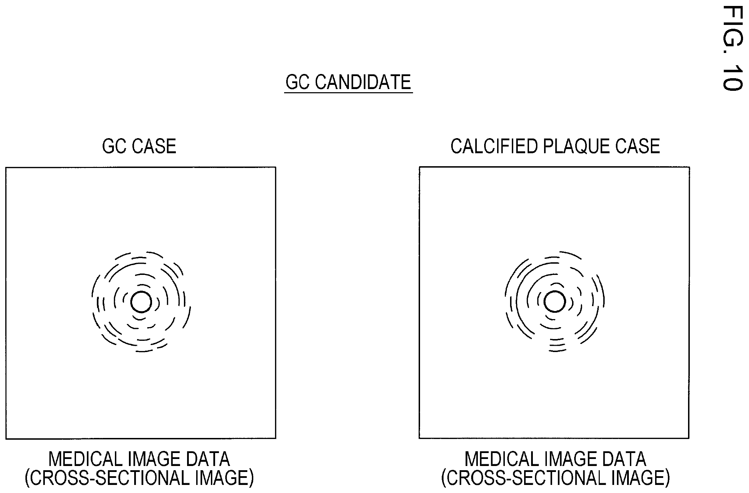

Fig. 10 is a diagram illustrating the guiding catheter and the calcified plaque on the cross-sectional image. Fig. 10 schematically illustrates the guiding catheter and the calcified plaque on the cross-sectional image, and may be different from an actual image. Since the ultrasound transmitted from the sensor is reflected off the inner perimeter surface of the guiding catheter, the region outside the guiding catheter has no high-luminance pixels and includes a set of relatively low-luminance pixels. In a case of calcified plaque, particularly in a case where calcification is seen on the entire perimeter, as with the guiding catheter, the ultrasound transmitted from the sensor is reflected off a portion of the calcified plaque, so that a region outside the calcified plaque has no high-luminance pixels and includes a set of relatively low-luminance pixels. It may be therefore difficult to distinguish between the guiding catheter and the calcified plaque on the cross-sectional image. Therefore, in the IVUS method, there is a possibility that the guiding catheter and the calcified lesion are erroneously detected. Hereinafter, the method of distinguishing between the guiding catheter and the calcified plaque will be described.

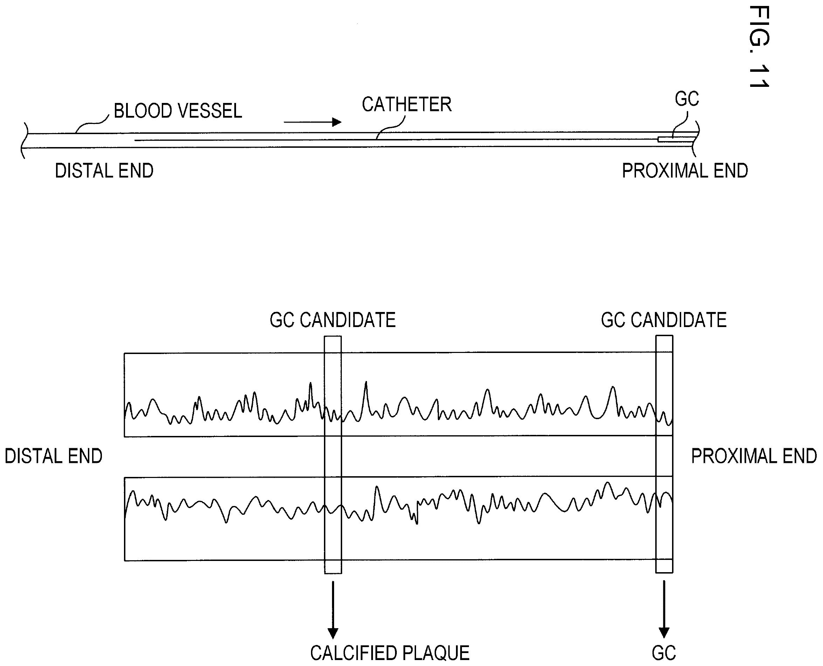

Fig. 11 is a diagram illustrating an example of the method of distinguishing between the guiding catheter and the calcified plaque. As illustrated in Fig. 11, during the pull-back operation, the distal end (sensor position) of the catheter is rotated in the circumferential direction while being moved from the distal end side to the proximal end side. The guiding catheter is inserted into the blood vessel from the proximal end side toward the distal end side. Therefore, the distal end of the catheter moves from the outside of the guiding catheter toward the proximal side of the guiding catheter, and moves from the entrance of the guiding catheter into the guiding catheter. The GC determination unit 55 can distinguish between the guiding catheter and the calcified plaque as follows.

That is, the GC determination unit 55 determines whether or not a guiding catheter candidate is present in the target frame on the basis of the pixel values of the guiding catheter determination region. In the example in Fig. 11, it is assumed that there are two guiding catheter candidates (GC candidates). In a case where the target frame is continuous from the proximal end of the blood vessel, the GC determination unit 55 can determine that the guiding catheter candidate is the guiding catheter. Furthermore, in a case where the target frame is not continuous from the proximal end of the blood vessel, the GC determination unit 55 can determine that the guiding catheter candidate is the calcified plaque.

It is possible to determine the presence or absence of the guiding catheter by selecting, one by one, the target frame sequentially from a target frame on the proximal end side up to a frame where it is determined that the guiding catheter is not present. In this case, since the target frames are sequentially selected frame by frame, the processing time becomes relatively long. On the other hand, as illustrated in Fig. 11, since the guiding catheter is inserted into the blood vessel from the proximal end side toward the distal end side, it is possible to shorten the processing time and achieve high-speed processing by quickly finding the entrance (end) of the guiding catheter. A method of selecting the target frame will be described below.

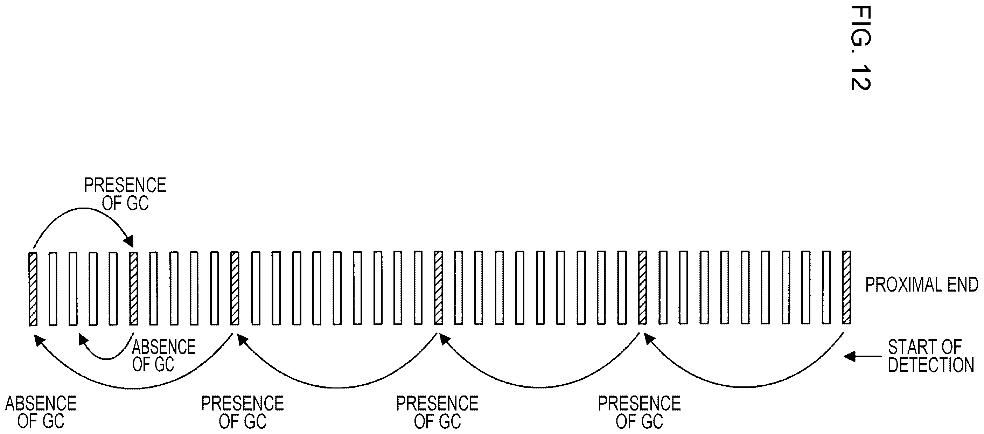

Fig. 12 is a diagram illustrating an example of the method of selecting the target frame for higher performance. As illustrated in Fig. 12, the target frame is selected at intervals of several frames from the proximal end side, and the presence or absence of the guiding catheter is determined in the selected target frame. In a case where the guiding catheter is present, another target frame is selected at an interval of several frames from the corresponding target frame, and the presence or absence of the guiding catheter is determined in the selected target frame. The same processing is repeatedly executed until a target frame where no guiding catheter is present. In a case where a target frame where no guiding catheter is present is found, another target frame is selected at an interval from the corresponding target frame toward the proximal end side. In this case, the interval is smaller. In a case where it is determined that the guiding catheter is present in the selected target frame, another target frame is selected at an interval from the corresponding target frame toward the distal end side. In this case, the interval is smaller. This makes it possible to identify the position of the entrance (end) of the guiding catheter.

As described above, the determination region identification unit 54 can identify the guiding catheter determination region on the basis of the target frames of cross-sectional images obtained by thinning out the plurality of frames of the acquired medical image data from the proximal end side, and the GC determination unit 55 can determine whether or not the guiding catheter is present in each target frame on the basis of the pixel values of the identified guiding catheter determination region.

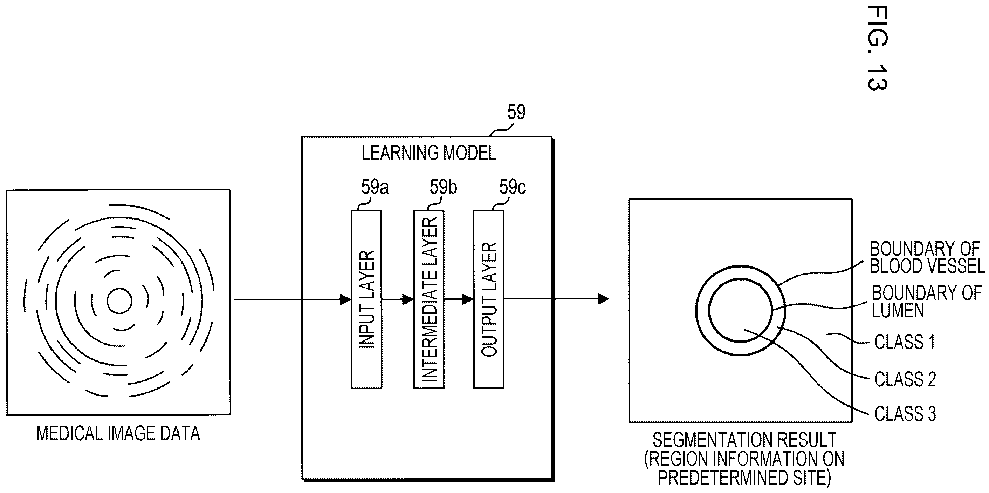

Fig. 13 is a diagram illustrating an example of processing based on the learning model 59. The learning model 59 includes an input layer 59a, an intermediate layer 59b, and an output layer 59c, and can be created as a U-Net, for example. The intermediate layer 59b includes a plurality of encoders and a plurality of decoders. The plurality of encoders repeat convolution processing on the medical image data input into the input layer 59a. The plurality of decoders repeat upsampling (deconvolution) processing on the image convolved by the encoders. When decoding the convolved image, processing of adding a feature map generated by the encoders to the image to be subjected to the deconvolution processing is executed. As a result, position information lost by the convolution processing can be retained, and more accurate segmentation (to which class each pixel belongs) can be output.

When the medical image data is input, the learning model 59 can output segmentation data (region information on a predetermined site). The segmentation data is obtained by classifying each pixel of the medical image data into a class. The learning model 59 can classify pixels of each piece of the input medical image data into, for example, three classes: classes 1, 2, and 3. Class 1 indicates Background that is a region outside the blood vessel. Class 2 indicates (Plaque + Media), that is a region of the blood vessel containing the plaque. Class 3 indicates Lumen that is the lumen of the blood vessel. Therefore, a boundary between pixels classified into Class 2 and pixels classified into Class 3 indicates a boundary of the lumen, and a boundary between pixels classified into Class 1 and pixels classified into Class 2 indicates a boundary of the blood vessel. That is, when the medical image data is input, the learning model 59 can output region information on a predetermined site of the blood vessel (that is, information indicating the boundary of the lumen and the boundary of the blood vessel). The predetermined site includes the boundary of the blood vessel and the boundary of the lumen. The region information is coordinate data of pixels indicating both the boundary of the lumen and the boundary of the blood vessel. Note that the learning model 59 is not limited to the U-Net, and may be, for example, a generative adversarial network (GAN), SegNet, or the like.

A method of generating the learning model 59 can be as follows. First, first training data including medical image data representing a cross-sectional image of a blood vessel and segmentation data indicating a class of each pixel of the medical image data is acquired. For example, the first training data may be collected and stored in the server 200 and acquired from the server 200. Next, the learning model 59 may be generated on the basis of the first training data to output segmentation data when the medical image data representing the cross-sectional image of a blood vessel is input into the learning model 59. In other words, the learning model 59 may be generated on the basis of the first training data to output region information on each of the boundary of a lumen and the boundary of a blood vessel when the medical image data representing the cross-sectional image of the blood vessel is input into the learning model 59.

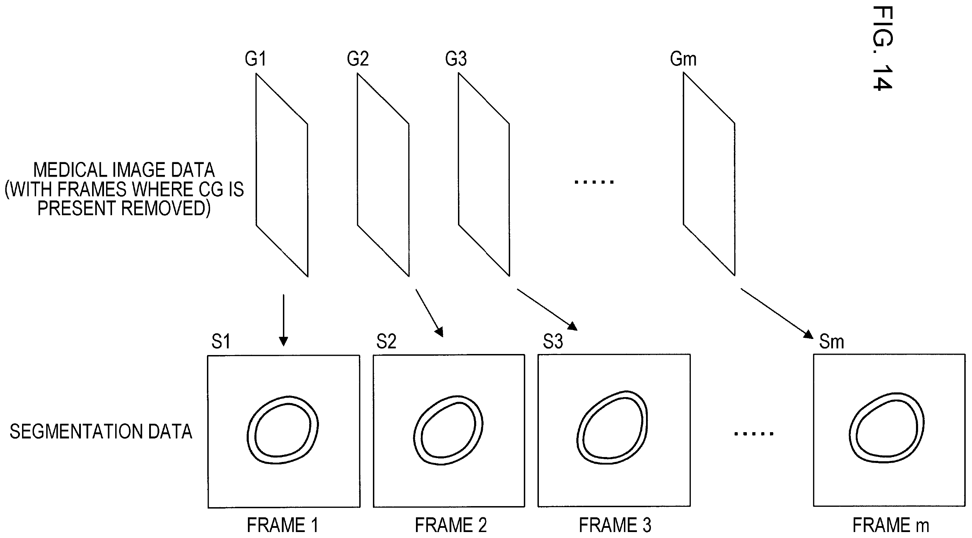

Fig. 14 is a diagram illustrating an example of the segmentation data output by the learning model 59. Medical image data (G1, G2, G3, ..., Gn) including a plurality of frames (frames 1 to n) of cross-sectional images is acquired from a plurality of time-series cross-sectional images of the blood vessel obtained as a result of one pull-back operation. Medical image data including frames of cross-sectional images remaining as a result of removing a target frame in which the guiding catheter is present from the plurality of frames of the acquired medical image data is denoted as (G1, G2, G3, ..., Gm). Here, m < n holds. The learning model 59 outputs segmentation data (S1, S2, S3, ..., Sm) corresponding to the frames 1 to m on a one-to-one basis. As described with reference to Fig. 13, each piece of segmentation data includes the region information on the boundary of the blood vessel and the boundary of the lumen.

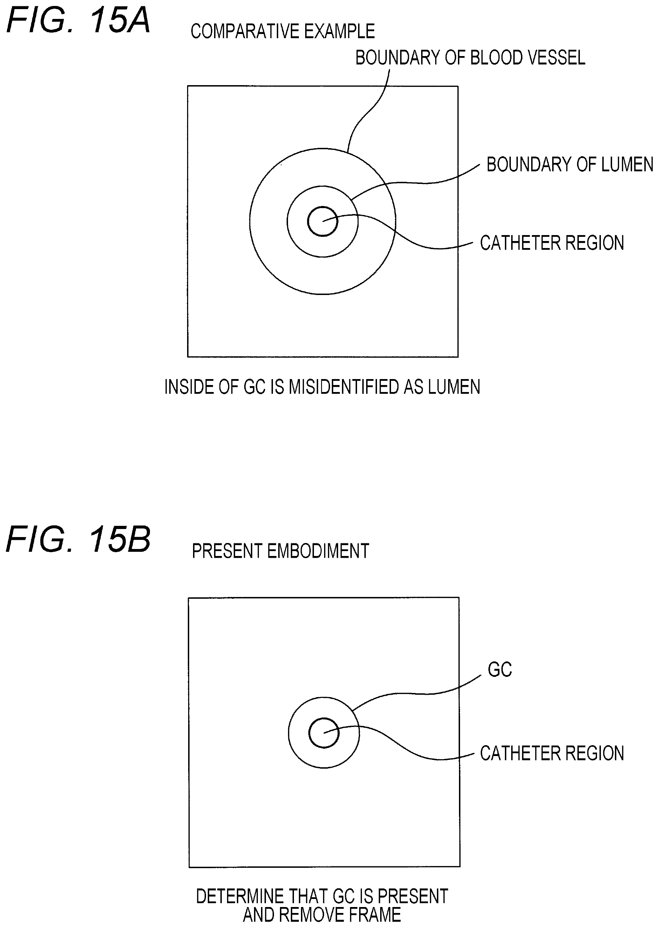

Fig. 15A is a diagram illustrating a comparative example where the guiding catheter is misidentified as the lumen of the blood vessel, and Fig. 15B is a diagram illustrating an example of detecting the guiding catheter according to the present embodiment. Fig. 15A illustrates a case where the determination region identification unit 54 and the GC determination unit 55 of the present embodiment are not included. In the comparative example, the guiding catheter is misidentified as the lumen of the blood vessel. Therefore, the position, diameter, area, and the like of a stenosis cannot be correctly evaluated.

On the other hand, as illustrated in Fig. 15B, according to the present embodiment, in a case where the guiding catheter is present in the blood vessel, the guiding catheter can be detected. The frame in which the guiding catheter is present can then be removed. That is, the control unit 51 can identify, using the learning model 59, the region information on the predetermined site of the blood vessel on the basis of the frames of cross-sectional images remaining as a result of removing the target frame in which the guiding catheter is present from the plurality of frames. It is therefore possible to correctly evaluate the position, diameter, area, and the like of the stenosis.

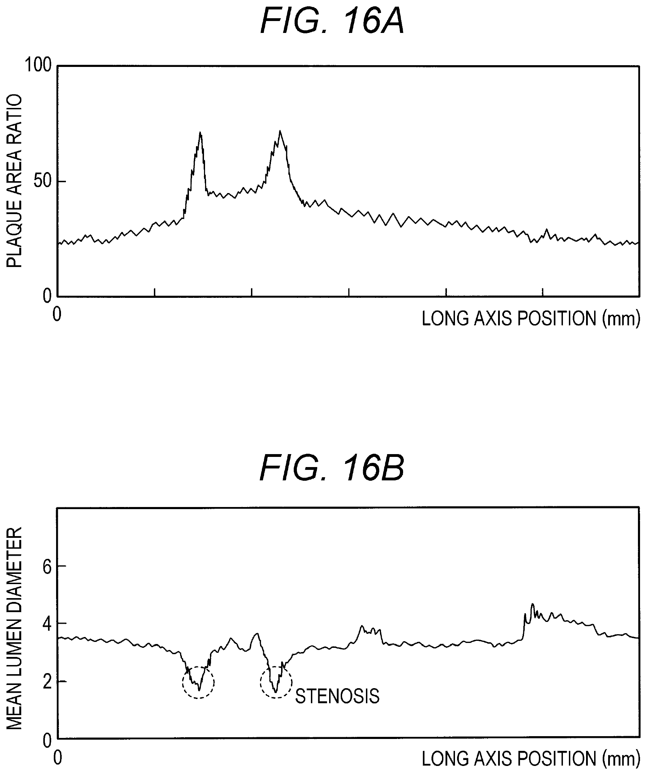

Figs. 16A and 16B are diagrams illustrating an example of blood vessel information obtained by the information processing apparatus 50. In Fig. 16A, the horizontal axis represents a long axis position of the blood vessel, and the vertical axis represents a plaque area ratio (plaque burden). The plaque area ratio illustrated in Fig. 16A is based on the segmentation data using only the frames remaining as a result of removing the target frame in which the guiding catheter is present, so that it is possible to correctly evaluate the position, diameter, area, and the like of the stenosis. It is possible to determine, by obtaining the blood vessel information as illustrated in Fig. 16A, at which position in the blood vessel a stent should be placed, how long the stent should be, and the like, for example.

In Fig. 16B, the horizontal axis represents the long axis position of the blood vessel, and the vertical axis represents a mean lumen diameter. As in Fig. 16A, the mean lumen diameter illustrated in Fig. 16B is based on the segmentation data using only the frames remaining as a result of removing the target frame in which the guiding catheter is present, so that it is possible to correctly evaluate the position, diameter, area, and the like of the stenosis. It is possible to determine, by obtaining the blood vessel information as illustrated in Fig. 16B, a stent diameter, for example.

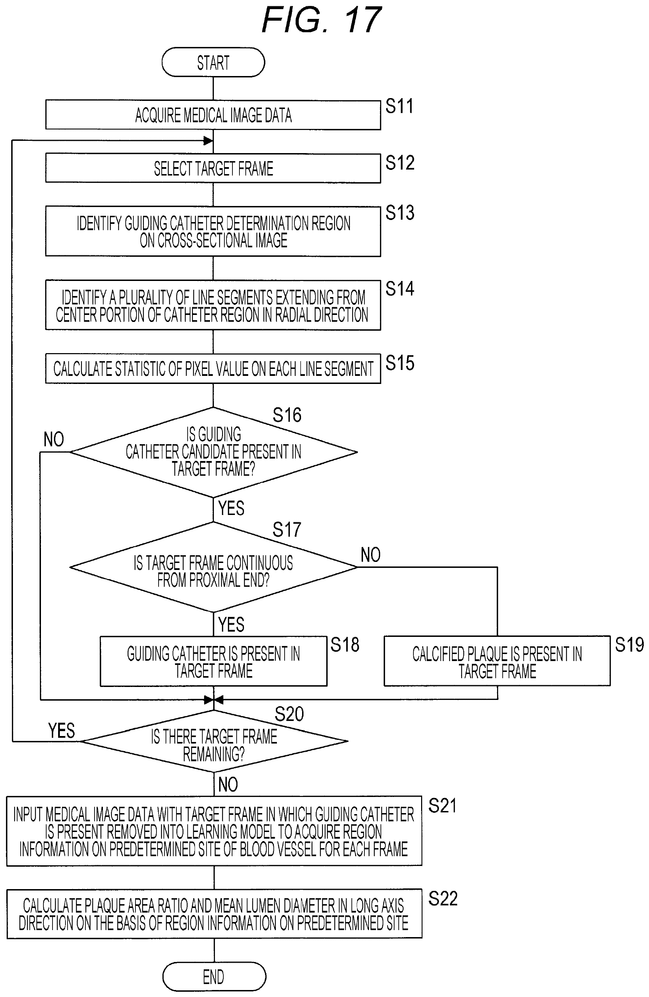

Fig. 17 is a diagram illustrating an example of a processing procedure by the information processing apparatus 50. For convenience, the following description will be given on the assumption that the control unit 51 takes the lead in executing the processing. The control unit 51 acquires medical image data (S11) and selects target frames (S12). The target frames may be selected frame by frame sequentially from the proximal end side, or may be selected by the method illustrated in Fig. 12. The control unit 51 identifies the guiding catheter determination region on each target frame of cross-sectional image (S13). As for the identification of the guiding catheter determination region, refer to Fig. 5 or Fig. 9.

The control unit 51 identifies a plurality of line segments (virtual line segments) extending from the center portion of the catheter region in the radial direction (S14), and calculates a statistic of pixel values on each line segment (S15). The statistic may be a mean, or may be a median or a mode. The control unit 51 determines whether or not a guiding catheter candidate is present in the target frame (S16).

In a case where the guiding catheter candidate is present in the target frame (YES in S16), the control unit 51 determines whether or not the target frame is a frame continuous from the proximal end (S17). In a case where the frame is continuous from the proximal end (YES in S17), the control unit 51 determines that the guiding catheter is present in the target frame (S18), and executes a process of step S20 to be described later.

In a case where the frame is not continuous from the proximal end (NO in S17), the control unit 51 determines that calcified plaque is present in the target frame (S19), and determines whether or not there is another target frame remaining (S20). In a case where no guiding catheter candidate is present in the target frame (NO in S16), the control unit 51 executes the process of step S20.

In a case where there is another target frame remaining (YES in S20), the control unit 51 continues the processes of step S12 and the subsequent steps. In a case where there is no target frame remaining (NO in S20), the control unit 51 inputs medical image data with the target frame in which the guiding catheter is present removed into the learning model 59 to acquire region information of a predetermined site of the blood vessel for each frame (S21), calculates a plaque area ratio and a mean lumen diameter in the long axis direction of the blood vessel on the basis of the acquired region information on the predetermined site (S22), and ends the processing.

A computer program of the present embodiment causes a computer to execute processing of: acquiring medical image data representing a plurality of frames of cross-sectional images of a luminal organ into which a guiding catheter has been inserted; identifying a guiding catheter determination region for determining the guiding catheter on the basis of target frames of cross-sectional images that are all or some of the plurality of frames of the acquired medical image data; and determining whether or not the guiding catheter is present in each of the target frames on a basis of pixel values of the identified guiding catheter determination region.

The computer program of the present embodiment causes the computer to further execute processing of identifying an inner perimeter boundary of the guiding catheter determination region on the basis of a catheter region of a catheter inserted into the guiding catheter and a diameter of the guiding catheter.

The computer program of the present embodiment causes the computer to further execute processing of: identifying a blood flow region on the basis of a difference between pixel values of corresponding positions on the plurality of target frames of cross-sectional images; and identifying an inner perimeter boundary of the guiding catheter determination region on the basis of the identified blood flow region.

The computer program of the present embodiment causes the computer to further execute processing of identifying an outer perimeter boundary of the guiding catheter determination region on the basis of a catheter region of a catheter inserted into the guiding catheter.

The computer program of the present embodiment causes the computer to further execute processing of: identifying an inner perimeter region portion having a diameter of an integral multiple of a predetermined distance in a radial direction from a center portion of a catheter region of a catheter inserted into the guiding catheter; and determining whether or not the guiding catheter is present in each of the target frames on the basis of pixel values of the guiding catheter determination region with the inner perimeter region portion removed.

The computer program of the present embodiment causes the computer to further execute processing of determining whether or not the guiding catheter is present in each of the target frames on the basis of pixel values of the guiding catheter determination region and pixel values on a plurality of line segments extending, in a radial direction, from a center portion of a catheter region of a catheter inserted into the guiding catheter.

The computer program of the present embodiment causes the computer to further execute processing of determining that, in a case where a statistic of the pixel values of the guiding catheter determination region in each of the target frames is less than or equal to a required threshold, the guiding catheter is present in the target frame.

The computer program of the present embodiment causes the computer to further execute processing of setting the required threshold on the basis of distribution of pixel values of a region inside an inner perimeter boundary of the guiding catheter determination region.

The computer program of the present embodiment causes the computer to further execute processing of: determining whether or not a guiding catheter candidate is present in each of the target frames on the basis of the pixel values of the guiding catheter determination region; determining that the guiding catheter candidate is the guiding catheter in a case where the target frame is continuous from a proximal end of the luminal organ; and determining that the guiding catheter candidate is a calcified plaque in a case where the target frame is not continuous from the proximal end of the luminal organ.

The computer program of the present embodiment causes the computer to further execute processing of identifying the guiding catheter determination region on the basis of target frames of cross-sectional images obtained by thinning out, from a proximal end side, the plurality of frames of the acquired medical image data.

The computer program of the present embodiment causes the computer to further execute processing of identifying region information on a predetermined site of the luminal organ on the basis of frames of cross-sectional images remaining as a result of removing a target frame in which the guiding catheter is present from the plurality of frames.

An information processing apparatus of the present embodiment includes: an acquisition unit that acquires medical image data representing a plurality of frames of cross-sectional images of a luminal organ into which a guiding catheter has been inserted; an identification unit that identifies a guiding catheter determination region for determining the guiding catheter on the basis of target frames of cross-sectional images that are all or some of the plurality of frames of the acquired medical image data; and a determination unit that determines whether or not the guiding catheter is present in each of the target frames on the basis of pixel values of the identified guiding catheter determination region.

An information processing method of the present embodiment includes: acquiring medical image data representing a plurality of frames of cross-sectional images of a luminal organ into which a guiding catheter has been inserted; identifying a guiding catheter determination region for determining the guiding catheter on the basis of target frames of cross-sectional images that are all or some of the plurality of frames of the acquired medical image data; and determining whether or not the guiding catheter is present in each of the target frames on the basis of pixel values of the identified guiding catheter determination region.

### Reference Signs List

- 1: Communication network
- 10: Catheter
- 20: MDU
- 30: Display apparatus
- 40: Input apparatus
- 50: Information processing apparatus
- 51: Control unit
- 52: Communication unit
- 53: Interface unit
- 54: Determination region identification unit
- 55: GC determination unit
- 56: Memory
- 57: Storage unit
- 58: Computer program
- 59: Learning model
- 59a: Input layer
- 59b: Intermediate layer
- 59c: Output layer
- 60: Recording medium reading unit
- 61: Recording medium
- 200: Server

## Claims

1. A computer program causing a computer to execute processing of:
acquiring medical image data (S11) representing a plurality of frames of cross-sectional images of a luminal organ into which a guiding catheter has been inserted;
identifying (S13) a guiding catheter determination region for determining the guiding catheter on a basis of target frames of cross-sectional images that are all or some of the plurality of frames of the medical image data acquired; and
determining whether or not the guiding catheter is present in each of the target frames on a basis of pixel values of the guiding catheter determination region identified.

2. The computer program according to claim 1, causing the computer to further execute processing of
identifying an inner perimeter boundary of the guiding catheter determination region on a basis of a catheter region of a catheter inserted into the guiding catheter and a diameter of the guiding catheter.

3. The computer program according to claim 1, causing the computer to further execute processing of:
identifying a blood flow region on a basis of a difference between pixel values of corresponding positions on the plurality of target frames of cross-sectional images; and
identifying an inner perimeter boundary of the guiding catheter determination region on a basis of the blood flow region identified.

4. The computer program according to any one of claims 1 to 3, causing the computer to further execute processing of
identifying an outer perimeter boundary of the guiding catheter determination region on a basis of a catheter region of a catheter inserted into the guiding catheter.

5. The computer program according to any one of claims 1 to 4, causing the computer to further execute processing of:
identifying an inner perimeter region portion having a diameter of an integral multiple of a predetermined distance in a radial direction from a center portion of a catheter region of a catheter inserted into the guiding catheter; and
determining whether or not the guiding catheter is present in each of the target frames on a basis of pixel values of the guiding catheter determination region with the inner perimeter region portion removed.

6. The computer program according to any one of claims 1 to 5, causing the computer to further execute processing of
determining whether or not the guiding catheter is present in each of the target frames on a basis of pixel values of the guiding catheter determination region and pixel values on a plurality of line segments extending, in a radial direction, from a center portion of a catheter region of a catheter inserted into the guiding catheter.

7. The computer program according to any one of claims 1 to 6, causing the computer to further execute processing of
determining that, in a case where a statistic of the pixel values of the guiding catheter determination region in each of the target frames is less than or equal to a required threshold, the guiding catheter is present in the target frame.

8. The computer program according to claim 7, causing the computer to further execute processing of
setting the required threshold on a basis of distribution of pixel values of a region inside an inner perimeter boundary of the guiding catheter determination region.

9. The computer program according to any one of claims 1 to 8, causing the computer to further execute processing of:
determining whether or not a guiding catheter candidate is present in each of the target frames on a basis of the pixel values of the guiding catheter determination region;
determining that the guiding catheter candidate is the guiding catheter in a case where the target frame is continuous from a proximal end of the luminal organ; and
determining that the guiding catheter candidate is a calcified plaque in a case where the target frame is not continuous from the proximal end of the luminal organ.

10. The computer program according to any one of claims 1 to 9, causing the computer to further execute processing of
identifying the guiding catheter determination region on a basis of target frames of cross-sectional images obtained by thinning out, from a proximal end side, the plurality of frames of the medical image data acquired.

11. The computer program according to any one of claims 1 to 10, causing the computer to further execute processing of
identifying region information on a predetermined site of the luminal organ on a basis of frames of cross-sectional images remaining as a result of removing a target frame in which the guiding catheter is present from the plurality of frames.

12. An information processing apparatus comprising:
an acquisition unit (52, 53) that acquires medical image data representing a plurality of frames of cross-sectional images of a luminal organ into which a guiding catheter has been inserted;
an identification unit (54) that identifies a guiding catheter determination region for determining the guiding catheter on a basis of target frames of cross-sectional images that are all or some of the plurality of frames of the medical image data acquired; and
a determination unit (55) that determines whether or not the guiding catheter is present in each of the target frames on a basis of pixel values of the guiding catheter determination region identified.

13. An information processing method comprising:
receiving (S11) medical image data representing a plurality of frames of cross-sectional images of a luminal organ into which a guiding catheter has been inserted;
identifying (S13) a guiding catheter determination region for determining the guiding catheter on a basis of target frames of cross-sectional images that are all or some of the plurality of frames of the medical image data acquired; and
determining whether or not the guiding catheter is present in each of the target frames on a basis of pixel values of the guiding catheter determination region identified.

## Patentansprüche

1. Computerprogramm, das einen Computer dazu veranlasst, eine Verarbeitung durchzuführen zum:
Erfassen medizinischer Bilddaten (S11), die eine Vielzahl der Einzelbilder von Querschnittsbildern eines luminalen Organs darstellen, in das ein Führungskatheter eingeführt wurde;
Identifizieren (S13) eines Bestimmungsbereichs für den Führungskatheter zur Bestimmung des Führungskatheters auf der Grundlage der Ziel-Einzelbilder von Querschnittsbildern, die alle oder einige von der Vielzahl der Einzelbilder der erfassten medizinischen Bilddaten darstellen; und
Bestimmen, auf der Grundlage von Pixelwerten des identifizierten Bestimmungsbereichs für den Führungskatheter, ob der Führungskatheter in jedem der Ziel-Einzelbilder vorhanden ist oder nicht.

2. Computerprogramm nach Anspruch 1, das den Computer dazu veranlasst, ferner eine Verarbeitung durchzuführen zum
Identifizieren einer inneren Umfangsgrenze des Bestimmungsbereichs für den Führungskatheter auf der Grundlage von einem Katheterbereich eines in den Führungskatheter eingeführten Katheters und von einem Durchmesser des Führungskatheters.

3. Computerprogramm nach Anspruch 1, das den Computer dazu veranlasst, ferner eine Verarbeitung durchzuführen zum:
Identifizieren eines Blutströmungsbereichs auf der Grundlage eines Unterschiedes zwischen den Pixelwerten entsprechender Positionen auf der Vielzahl der Ziel-Einzelbilder von Querschnittsbildern; und
Identifizieren einer inneren Umfangsgrenze des Bestimmungsbereichs für den Führungskatheter auf der Grundlage des identifizierten Blutströmungsbereichs.

4. Computerprogramm nach einem der Ansprüche 1 bis 3, das den Computer dazu veranlasst, eine Verarbeitung durchzuführen zum:
Identifizieren einer äußeren Umfangsgrenze des Bestimmungsbereichs für den Führungskatheter auf der Grundlage eines Katheterbereichs eines in den Führungskatheter eingeführten Katheters.

5. Computerprogramm nach einem der Ansprüche 1 bis 4, das den Computer dazu veranlasst, eine Verarbeitung durchzuführen zum:
Identifizieren eines inneren Umfangsbereichsabschnitts, der einen Durchmesser aufweist, welcher ein ganzzahliges Vielfaches eines vorbestimmten Abstands in einer radialen Richtung von einem Mittelabschnitt eines Katheterbereichs eines Katheters beträgt, der in den Führungskatheter eingeführt ist; und
Bestimmen, ob der Führungskatheter in jedem der Ziel-Einzelbilder vorhanden ist oder nicht, auf der Grundlage von Pixelwerten des identifizierten Bestimmungsbereichs für den Führungskatheter, wobei der innere Umfangsbereichsabschnitt entfernt wurde.

6. Computerprogramm nach einem der Ansprüche 1 bis 5, das den Computer dazu veranlasst, ferner eine Verarbeitung durchzuführen zum
Bestimmen, ob der Führungskatheter in jedem der Ziel-Einzelbilder vorhanden ist oder nicht, auf der Grundlage von Pixelwerten des Bestimmungsbereichs für den Führungskatheter und von Pixelwerten auf einer Vielzahl von Liniensegmenten, die sich in einer radialen Richtung von einem Mittelabschnitt eines Katheterbereichs eines Katheters erstrecken, der in den Führungskatheter eingeführt wurde.

7. Computerprogramm nach einem der Ansprüche 1 bis 6, das den Computer dazu veranlasst, ferner eine Verarbeitung durchzuführen zum:
Bestimmen, dass in einem Fall, in dem eine Statistik der Pixelwerte des Bestimmungsbereichs für den Führungskatheter in jedem der Ziel-Einzelbilder kleiner oder gleich einem erforderlichen Schwellenwert ist, der Führungskatheter in dem Ziel-Einzelbild vorhanden ist.

8. Computerprogramm nach Anspruch 7, das den Computer dazu veranlasst, ferner eine Verarbeitung durchzuführen zum:
Festlegen des erforderlichen Schwellenwertes auf der Grundlage der Verteilung von Pixelwerten eines Bereichs innerhalb einer inneren Umfangsgrenze des Bestimmungsbereichs für den Führungskatheter.

9. Computerprogramm nach einem der Ansprüche 1 bis 8, das den Computer dazu veranlasst, ferner eine Verarbeitung durchzuführen zum:
Bestimmen, auf der Grundlage von den Pixelwerten des Bestimmungsbereichs für den Führungskatheter, ob ein Führungskatheter-Kandidat in jedem der Ziel-Einzelbilder vorhanden ist oder nicht;
Feststellen, dass der Führungskatheter-Kandidat der Führungskatheter ist, in einem Fall, in welchem das Ziel-Einzelbild von dem proximalen Ende des luminalen Organs kontinuierlich ist; und
Feststellen, dass der Führungskatheter-Kandidat ein kalzifizierter Plaque ist, in einem Fall, in welchem das Ziel-Einzelbild von dem proximalen Ende des luminalen Organs nicht kontinuierlich ist.

10. Computerprogramm nach einem der Ansprüche 1 bis 9, das den Computer dazu veranlasst, ferner eine Verarbeitung durchzuführen zum:
Identifizieren des Bestimmungsbereichs des Führungskatheters auf der Grundlage der Ziel-Einzelbilder von Querschnittsbildern, die durch Ausdünnen der Vielzahl von Einzelbildern der erfassten medizinischen Bilddaten von der proximalen Endseite her erhalten wurden.

11. Computerprogramm nach einem der Ansprüche 1 bis 10, das den Computer dazu veranlasst, ferner eine Verarbeitung durchzuführen zum:
Identifizieren von Bereichsinformationen an einer vorbestimmten Stelle des luminalen Organs auf der Grundlage der Einzelbilder von Querschnittsbildern, die als Ergebnis des Entfernens eines Ziel-Einzelbildes, in welchem der Führungskatheter vorhanden ist, aus der Vielzahl von Bildern verbleiben.

12. Informationsverarbeitungsvorrichtung, umfassend:
eine Erfassungseinheit (52, 53), die medizinische Bilddaten (S11) erfasst, welche eine Vielzahl der Einzelbilder von Querschnittsbildern eines luminalen Organs darstellen, in das ein Führungskatheter eingeführt wurde;
eine Identifizierungseinheit (54), die einen Bestimmungsbereich für den Führungskatheter zur Bestimmung des Führungskatheters auf der Grundlage der Ziel-Einzelbilder von Querschnittsbildern identifiziert, die alle oder einige von der Vielzahl der Einzelbilder der erfassten medizinischen Bilddaten darstellen; und
eine Bestimmungseinheit (55), die auf der Grundlage von Pixelwerten des identifizierten Bestimmungsbereichs für den Führungskatheter bestimmt, ob der Führungskatheter in jedem der Ziel-Einzelbilder vorhanden ist oder nicht.

13. Informationsverarbeitungsverfahren, umfassend:
Empfangen (S11) von medizinischen Bilddaten, die eine Vielzahl der Einzelbilder von Querschnittsbildern eines luminalen Organs darstellen, in das ein Führungskatheter eingeführt wurde;
Identifizieren (S13) eines Bestimmungsbereichs für den Führungskatheter zur Bestimmung des Führungskatheters auf der Grundlage der Ziel-Einzelbilder von Querschnittsbildern, die alle oder einige von der Vielzahl der Einzelbilder der erfassten medizinischen Bilddaten darstellen; und
Bestimmen, auf der Grundlage von Pixelwerten des identifizierten Bestimmungsbereichs für den Führungskatheter, ob der Führungskatheter in jedem der Ziel-Einzelbilder vorhanden ist oder nicht.

## Revendications

1. Programme informatique permettant à un ordinateur d'exécuter le traitement :
d'acquisition de données d'images médicales (S11) représentant une pluralité de trames de données d'images en coupe transversale d'un organe luminal dans lequel un cathéter de guidage a été inséré ;
d'identification (S13) d'une zone de détermination de cathéter de guidage pour déterminer le cathéter de guidage sur la base de trames cibles d'images en coupe transversale qui représentent la totalité ou une partie de la pluralité de trames des données d'images médicales acquises ; et
de détermination si le cathéter de guidage est présent ou non dans chacune des trames cibles sur la base des valeurs de pixels de la zone de détermination du cathéter de guidage identifiée.

2. Programme informatique selon la revendication 1, amenant l'ordinateur à exécuter en outre un traitement d'identification d'une limite de périmètre interne de la zone de détermination de cathéter de guidage sur la base d'une zone de cathéter d'un cathéter inséré dans le cathéter de guidage et d'un diamètre du cathéter de guidage.

3. Programme informatique selon la revendication 1, amenant l'ordinateur à exécuter en outre le traitement :
d'identification d'une zone d'écoulement sanguin sur la base d'une différence entre des valeurs de pixels de positions correspondantes sur la pluralité de trames d'images cibles en coupe transversale ; et
l'identification d'une limite périmétrique interne de la zone de détermination du cathéter de guidage sur la base de la zone d'écoulement sanguin identifiée.

4. Programme informatique selon l'une quelconque des revendications 1 à 3, amenant l'ordinateur à exécuter en outre un traitement
d'identification d'une limite périmétrique externe de la zone de détermination de cathéter de guidage sur la base d'une zone de cathéter d'un cathéter inséré dans le cathéter de guidage.

5. Programme informatique selon l'une quelconque des revendications 1 à 4, amenant l'ordinateur à exécuter en outre un traitement :
d'identification d'une partie de zone périmétrique interne ayant un diamètre représentant un multiple entier d'une distance prédéterminée dans une direction radiale à partir d'une partie centrale d'une zone de cathéter d'un cathéter inséré dans le cathéter de guidage ; et
de détermination si le cathéter de guidage est présent ou non dans chacune des trames cibles sur la base des valeurs de pixels de la zone de détermination du cathéter de guidage, la partie de zone périmétrique interne étant retirée.

6. Programme informatique selon l'une quelconque des revendications 1 à 5, amenant l'ordinateur à exécuter en outre un traitement
de détermination si oui ou non le cathéter de guidage est présent dans chacun des trames cibles sur la base de valeurs de pixels de la zone de détermination de cathéter de guidage et de valeurs de pixels sur une pluralité de segments de ligne s'étendant, dans une direction radiale, à partir d'une partie centrale d'une zone de cathéter d'un cathéter inséré dans le cathéter de guidage.

7. Programme informatique selon l'une quelconque des revendications 1 à 6, amenant l'ordinateur à exécuter en outre un traitement
de détermination que, dans un cas où une statistique des valeurs de pixels de la zone de détermination de cathéter de guidage dans chacune des trames cibles est inférieure ou égale à un seuil requis, le cathéter de guidage est présent dans la trame cible.

8. Programme informatique selon la revendication 7, amenant l'ordinateur à exécuter en outre un traitement de
réglage du seuil requis sur la base d'une répartition de valeurs de pixels d'une zone à l'intérieur d'une limite de périmètre interne de la zone de détermination de cathéter de guidage.

9. Programme informatique selon l'une quelconque des revendications 1 à 8, amenant l'ordinateur à exécuter en outre un traitement de :
détermination si un cathéter de guidage candidat est présent ou non dans chacune des trames cibles sur la base des valeurs de pixels de la zone de détermination du cathéter-guide ;
détermination que le cathéter de guidage candidat est le cathéter de guidage dans un cas où la trame cible est continue à partir d'une extrémité proximale de l'organe luminal ; et
détermination que le cathéter de guidage candidat est une plaque calcifiée dans un cas où la trame cible n'est pas continue à partir de l'extrémité proximale de l'organe luminal.

10. Programme informatique selon l'une quelconque des revendications 1 à 9, amenant l'ordinateur à exécuter en outre un traitement
d'identification de la zone de détermination de cathéter de guidage sur la base de trames cibles d'images en coupe transversale obtenues en amincissant, à partir d'un côté d'extrémité proximale, la pluralité de trames des données d'image médicale acquises.

11. Programme informatique selon l'une quelconque des revendications 1 à 10, amenant l'ordinateur à exécuter en outre un traitement
d'identification d'informations de zone sur un site prédéterminé de l'organe luminal sur la base de trames d'images en coupe transversale restantes résultant du retrait d'une trame cible dans laquelle le cathéter de guidage est présent à partir de la pluralité de trames.

12. Appareil de traitement de l'information comprenant :
une unité d'acquisition (52, 53) qui acquiert des données d'images médicales représentant une pluralité de trames d'images en coupe transversale d'un organe luminal dans lequel un cathéter de guidage a été inséré ;
une unité d'identification (54) qui identifie une zone de détermination de cathéter de guidage pour déterminer le cathéter de guidage sur la base de trames cibles d'images en coupe transversale qui sont tout ou partie de la pluralité de trames des données d'images médicales acquises ; et
une unité de détermination qui détermine si le cathéter de guidage est présent ou non dans chacune des trames cibles sur la base des valeurs de pixels de la zone de détermination du cathéter de guidage identifiée.

13. Procédé de traitement d'informations comprenant :
la réception (S11) de données d'images médicales représentant une pluralité de trames d'images en coupe transversale d'un organe luminal dans lequel un cathéter de guidage a été inséré ;
l'identification (S13) d'une zone de détermination de cathéter de guidage permettant de déterminer le cathéter de guidage sur la base de trames d'images cibles en coupe transversale qui représentent la totalité ou une partie de la pluralité de trames des données d'images médicales acquises ; et
la détermination si le cathéter de guidage est présent ou non dans chacune des trames cibles sur la base des valeurs de pixels de la zone de détermination du cathéter de guidage identifiée.
